# EUROPEAN PATENT APPLICATION

(11) **EP 4 763 069 A1**
(43) Date of publication of application: **24.06.2026**
(21) Application number: 25224683.0
(22) Date of filing: 17.12.2025
(51) Int. Cl.: A61B 5/024, A61B 5/389, A61B 5/00, G06F 1/16

(54) **WRIST-WEARABLE DEVICE WITH INTEGRATED ELECTROMYOGRAPHY SENSOR AND HEART RATE MONITOR**

(30) Priority: 19.12.2024 US 202463736539 P; 11.12.2025 US 202519417275
(71) Applicant: Meta Platforms Technologies, LLC, Menlo Park, CA 94025 (US)
(72) Inventor: Dahlgren, Aron, Menlo Park (US); Lu, Cunyou, Menlo Park (US); Neergaard, Samer, Menlo Park (US); Zhao, Wancheng, Menlo Park (US); Kowalski, Stefan Ahldor, Menlo Park (US); Auclair, Martin, Menlo Park (US); Duan, Xiyu, Menlo Park (US); Lee, Ricky Chin, Menlo Park (US); Yuen, Samuel, Menlo Park (US); Keane, Ciaran Joseph, Menlo Park (US); Li, Xinhui, Menlo Park (US)
(74) Representative: Murgitroyd & Company

(57) **Abstract**

An example a wrist-wearable device, comprises a capsule that includes an electromyography (EMG) sensor configured to provide biopotential information of a user for determining a movement of the user. The capsule includes a photoplethysmogram (PPG) sensor configured to provide information for determining a heart rate of the user. The biopotential information and information for determining the heart rate of the user is received simultaneously, and sensing of the biopotential information does not alter the information for determining heart rate of the user, and sensing of the information for determining the heart rate of the user does not alter the biopotential information.

## Description

### TECHNICAL FIELD

This relates generally to packaging a heart rate monitor (HRM) and a biopotential sensor within a housing of a wrist-wearable device, where the data from the HRM and the biopotential sensor are used to interact with an extended-reality environment.

### BACKGROUND

In some applications, heart rate monitors and biopotential sensors (e.g., electromyography sensors) are independently used for collecting data of users. However, these sensors are provided by separate devices. Having to wear multiple devices can be cumbersome for the user and may also cause the user to just don a single device, which can degrade user experience. In some circumstances these separate devices might also compete for the same location on the wearer's body (e.g. a wrist) and can force the user to only be able to use one device at a time.

As such, there is a need to address one or more of the above-identified challenges. A brief summary of solutions to the issues noted above are described below.

### SUMMARY

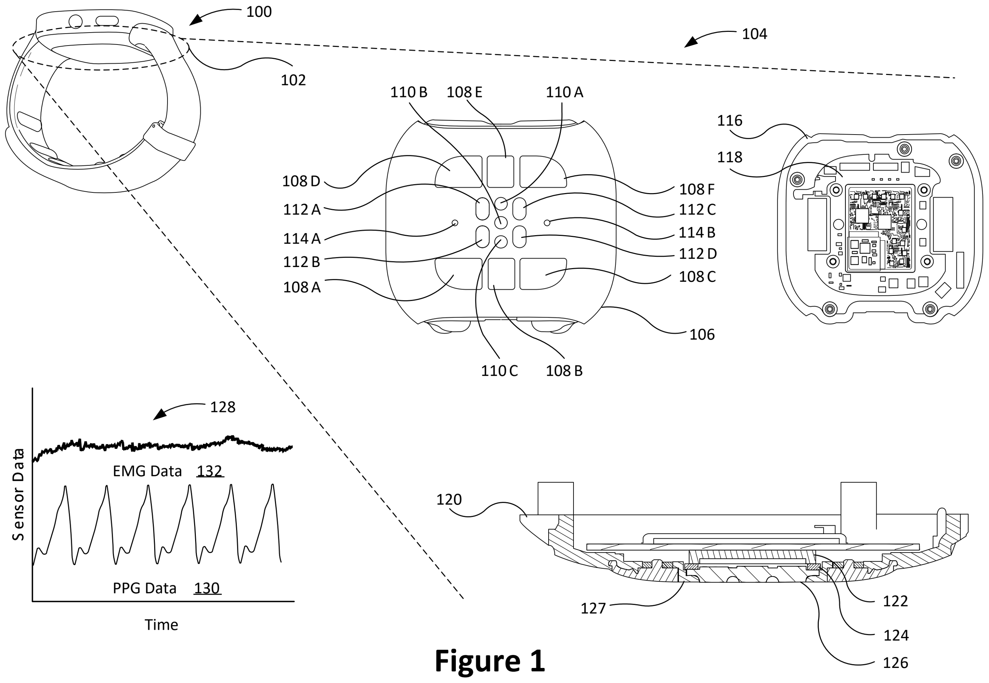

As such, there is a need for a single device that incorporates a heart rate monitor and a biopotential sensor such that the user can have a full user experience without compromising comfort and convenience. An example wrist-wearable device is described herein that resolves the concerns noted in the background section. The example wrist-wearable device can comprise a capsule that includes: an electromyography (EMG) sensor configured to provide biopotential information of a user for determining a movement of the user, and a photoplethysmogram (PPG) sensor configured to provide information for determining a heart rate of the user. For example, Figure 1 shows a wrist-wearable device 100 that includes a heart rate monitor (HRM), e.g., a PPG based heart rate monitor, and an electromyography (EMG) sensor. The biopotential information and information for determining the heart rate of the user is received simultaneously, and sensing of the biopotential information does not alter the information for determining the heart rate of the user, and sensing of the information for determining the heart rate of the user does not alter the biopotential information. For example, Figure 1 shows a chart 128 that illustrates the data being received from the PPG sensor is not degraded while the data from the EMG sensor is being received, and vice versa.

The devices and/or systems described herein can be configured to include instructions that cause the performance of methods and operations associated with the presentation and/or interaction with an extended-reality (XR) headset. These methods and operations can be stored on a non-transitory computer-readable storage medium of a device or a system. It is also noted that the devices and systems described herein can be part of a larger, overarching system that includes multiple devices. A non-exhaustive list of electronic devices that can, either alone or in combination (e.g., a system), include instructions that cause the performance of methods and operations associated with the presentation and/or interaction with an XR experience include an extended-reality headset (e.g., a mixed-reality (MR) headset or an augmented-reality (AR) headset as two examples), a wrist-wearable device, an intermediary processing device, a smart textile-based garment, etc. For example, when an XR headset is described, it is understood that the XR headset can be in communication with one or more other devices (e.g., a wrist-wearable device, a server, intermediary processing device) which together can include instructions for performing methods and operations associated with the presentation and/or interaction with an extended-reality system (i.e., the XR headset would be part of a system that includes one or more additional devices). Multiple combinations with different related devices are envisioned, but not recited for brevity.

According to an aspect, there is provided a wrist-wearable device, comprising:
a wearable capsule, wherein the wearable capsule includes a bottom capsule portion configured to contact a user;
an electromyography, EMG, sensor disposed within the wearable capsule, the EMG sensor configured to provide biopotential information of a user for determining a movement of the user; and
a photoplethysmogram, PPG, sensor disposed within the wearable capsule, the PPG sensor configured to provide information for determining a heart rate of the user, wherein the biopotential information and information for determining the heart rate of the user is received simultaneously, and sensing of the biopotential information does not alter the information for determining heart rate of the user, and sensing of the information for determining the heart rate of the user does not alter the biopotential information.

In one embodiment, the wrist-wearable device further comprises:
a plurality of electrodes coupled to the bottom capsule portion and electrically connected to the EMG sensor, wherein the plurality of electrodes is configured to permit transmission of biopotential information from the user to the EMG sensor.

In one embodiment, the plurality of electrodes is integrated with the bottom capsule portion.

In one embodiment, a central portion of the bottom capsule portion coupled to the plurality of electrodes is raised relative to a surrounding portion of the bottom capsule portion.

In one embodiment, the plurality of electrodes is sealingly coupled to the bottom capsule portion.

In one embodiment, the wrist-wearable device further comprises a sealing gasket disposed between an electrode of the plurality of electrodes and the bottom capsule portion.

In one embodiment, the wrist-wearable device further comprises a printed circuit board disposed within the wearable capsule, wherein the EMG sensor and the PPG sensor are coupled to the printed circuit board.

In one embodiment, the wrist-wearable device further comprises a fastener extending through the printed circuit board removably coupled to an electrode of the plurality of electrodes, wherein the fastener is configured to couple the printed circuit board to the wearable capsule.

In one embodiment, the printed circuit board includes power line interference shielding.

In one embodiment, the wrist-wearable device further comprises:
a plurality of light pipes coupled to the PPG sensor and the bottom capsule portion, wherein the plurality of light pipes is configured to permit transmission of light between the PPG sensor and the bottom capsule portion.

In one embodiment, the plurality of light pipes is integrated with the bottom capsule portion.

In one embodiment, the plurality of light pipes includes an outlet light pipe configured to transmit light from an outlet portion of PPG sensor to the user and an inlet light pipe configured to transmit light from the user to an inlet portion of the PPG sensor.

In one embodiment, the wrist-wearable device further comprises:
an optical sealing gasket disposed between the outlet portion of the PPG sensor and the inlet portion of the PPG sensor, wherein the optical sealing gasket is configured to reduce crosstalk between the outlet portion and the inlet portion.

In one embodiment, the optical sealing gasket includes:
a compressible foam layer; and
a pressure-sensitive adhesive layer coupled to the compressible foam layer.

In one embodiment, the bottom capsule portion comprises polycarbonate or polybutylene terephthalate.

According to another aspect, there is provided a wrist-wearable device, comprising:
a wearable capsule, wherein the wearable capsule includes a bottom capsule portion configured to contact a user;
an electromyography, EMG, sensor disposed within the wearable capsule, the EMG sensor configured to provide biopotential information of a user for determining a movement of the user;
a plurality of electrodes coupled to the bottom capsule portion and electrically connected to the EMG sensor, wherein the plurality of electrodes is configured to permit transmission of biopotential information from the user to the EMG sensor;
a photoplethysmogram, PPG, sensor disposed within the wearable capsule, the PPG sensor configured to provide information for determining a heart rate of the user; and
a plurality of light pipes coupled to the PPG sensor and the bottom capsule portion, wherein the plurality of light pipes is configured to permit the transmission of light between the PPG sensor and the bottom capsule portion,
wherein the biopotential information and information for determining the heart rate of the user is received simultaneously, and sensing of the biopotential information does not alter the information for determining heart rate of the user, and sensing of the information for determining the heart rate of the user does not alter the biopotential information.

In one embodiment, the plurality of electrodes and the plurality of light pipes are each integrated with the bottom capsule portion.

In one embodiment, the wrist-wearable device further comprises:
an optical sealing gasket comprising:
   a compressible foam layer; and
   a pressure-sensitive adhesive layer coupled to the compressible foam layer, wherein the optical sealing gasket is disposed between an outlet portion of the PPG sensor and an inlet portion of the PPG sensor,
wherein the optical sealing gasket is configured to reduce crosstalk between the outlet portion and the inlet portion and reduce compressive force applied to the plurality of electrodes.

According to a further aspect, there is provided a method to manufacture a wrist-wearable device, the method comprising:
injecting a first material into a mold to form a first shot of a bottom capsule portion of the wrist-wearable device, wherein the first shot of the bottom capsule portion includes a plurality of light pipes configured to permit transmission of light between a photoplethysmogram, PPG, sensor and the bottom capsule portion;
positioning a plurality of electrodes relative to the first shot of the bottom capsule portion of the wrist-wearable device, wherein the plurality of electrodes is configured to permit transmission of biopotential information from a user to an electromyography, EMG, sensor; and
injecting a second material into the mold to form a second shot of the bottom capsule portion of the wrist-wearable device, wherein the second shot of the bottom capsule portion bonds the plurality of electrodes to the bottom capsule portion of the wrist-wearable device.

In one embodiment, the method further comprises:
applying a hard coat to the bottom capsule portion and the plurality of electrodes; and
removing the hard coat from the plurality of electrodes after applying the hard coat to the bottom capsule portion.

It will be appreciated that any features described herein as being suitable for incorporation into one or more aspects or embodiments of the present disclosure are intended to be generalizable across any and all aspects and embodiments of the present disclosure. Other aspects of the present disclosure can be understood by those skilled in the art in light of the description, the claims, and the drawings of the present disclosure. The foregoing general description and the following detailed description are exemplary and explanatory only and are not restrictive of the claims.

### BRIEF DESCRIPTION OF THE DRAWINGS

For a better understanding of the various described embodiments, reference should be made to the Detailed Description below, in conjunction with the following drawings in which like reference numerals refer to corresponding parts throughout the figures.
Figure 1 illustrates a wrist-wearable device that includes a heart rate monitor (HRM) and an electromyography (EMG) sensor, in accordance with some embodiments.
Figure 2 illustrates integration of electrodes of an EMG sensor with a bottom capsule portion of a wrist-wearable device, in accordance with some embodiments.
Figure 3 illustrates a PPG assembly that uses an optical sealing gasket to reduce light bleed between the emitters and the receivers, in accordance with some embodiments.
Figure 4 illustrates a method of applying a hard coat to a surface of the wrist-wearable device while ensuring skin contact portions of the electrodes are not impeded, in accordance with some embodiments.
Figure 5 illustrates a method of limiting plastic overflow when overmolding over the electrodes in the bottom cover of a wrist-wearable device, in accordance with some embodiments.
Figures 6A, 6B, 6C-1, and 6C-2 illustrate example MR and AR systems, in accordance with some embodiments.

In accordance with common practice, the various features illustrated in the drawings may not be drawn to scale. Accordingly, the dimensions of the various features may be arbitrarily expanded or reduced for clarity. In addition, some of the drawings may not depict all of the components of a given system, method, or device. Finally, like reference numerals may be used to denote like features throughout the specification and figures.

### DETAILED DESCRIPTION

Numerous details are described herein to provide a thorough understanding of the example embodiments illustrated in the accompanying drawings. However, some embodiments may be practiced without many of the specific details, and the scope of the claims is only limited by those features and aspects specifically recited in the claims. Furthermore, well-known processes, components, and materials have not necessarily been described in exhaustive detail so as to avoid obscuring pertinent aspects of the embodiments described herein.

### Overview

Embodiments of this disclosure can include or be implemented in conjunction with various types of extended-realities (XRs) such as mixed-reality (MR) and augmented-reality (AR) systems. MRs and ARs, as described herein, are any superimposed functionality and/or sensory-detectable presentation provided by MR and AR systems within a user's physical surroundings. Such MRs can include and/or represent virtual realities (VRs) and VRs in which at least some aspects of the surrounding environment are reconstructed within the virtual environment (e.g., displaying virtual reconstructions of physical objects in a physical environment to avoid the user colliding with the physical objects in a surrounding physical environment). In the case of MRs, the surrounding environment that is presented through a display is captured via one or more sensors configured to capture the surrounding environment (e.g., a camera sensor, time-of-flight (ToF) sensor). While a wearer of an MR headset can see the surrounding environment in full detail, they are seeing a reconstruction of the environment reproduced using data from the one or more sensors (i.e., the physical objects are not directly viewed by the user). An MR headset can also forgo displaying reconstructions of objects in the physical environment, thereby providing a user with an entirely VR experience. An AR system, on the other hand, provides an experience in which information is provided, e.g., through the use of a waveguide, in conjunction with the direct viewing of at least some of the surrounding environment through a transparent or semi-transparent waveguide(s) and/or lens(es) of the AR headset. Throughout this application, the term "extended reality (XR)" is used as a catchall term to cover both ARs and MRs. In addition, this application also uses, at times, a head-wearable device or headset device as a catchall term that covers XR headsets such as AR headsets and MR headsets.

As alluded to above, an MR environment, as described herein, can include, but is not limited to, non-immersive, semi-immersive, and fully immersive VR environments. As also alluded to above, AR environments can include marker-based AR environments, markerless AR environments, location-based AR environments, and projection-based AR environments. The above descriptions are not exhaustive and any other environment that allows for intentional environmental lighting to pass through to the user would fall within the scope of an AR, and any other environment that does not allow for intentional environmental lighting to pass through to the user would fall within the scope of an MR.

The AR and MR content can include video, audio, haptic events, sensory events, or some combination thereof, any of which can be presented in a single channel or in multiple channels (such as stereo video that produces a three-dimensional effect to a viewer). Additionally, AR and MR can also be associated with applications, products, accessories, services, or some combination thereof, which are used, for example, to create content in an AR or MR environment and/or are otherwise used in (e.g., to perform activities in) AR and MR environments.

Interacting with these AR and MR environments described herein can occur using multiple different modalities and the resulting outputs can also occur across multiple different modalities. In one example AR or MR system, a user can perform a swiping in-air hand gesture to cause a song to be skipped by a song-providing application programming interface (API) providing playback at, for example, a home speaker.

A hand gesture, as described herein, can include an in-air gesture, a surface-contact gesture, and/or other gestures that can be detected and determined based on movements of a single hand (e.g., a one-handed gesture performed with a user's hand that is detected by one or more sensors of a wearable device (e.g., electromyography (EMG) and/or inertial measurement units (IMUs) of a wrist-wearable device, and/or one or more sensors included in a smart textile-wearable device) and/or detected via image data captured by an imaging device of a wearable device (e.g., a camera of a head-wearable device, an external tracking camera setup in the surrounding environment)). "In-air" generally includes gestures in which the user's hand does not contact a surface, object, or portion of an electronic device (e.g., a head-wearable device or other communicatively coupled device, such as the wrist-wearable device), in other words the gesture is performed in open air in 3D space and without contacting a surface, an object, or an electronic device. Surface-contact gestures (contacts at a surface, object, body part of the user, or electronic device) more generally are also contemplated in which a contact (or an intention to contact) is detected at a surface (e.g., a single- or double-finger tap on a table, on a user's hand or another finger, on the user's leg, on a couch, on a steering wheel). The different hand gestures disclosed herein can be detected using image data and/or sensor data (e.g., neuromuscular signals sensed by one or more biopotential sensors (e.g., EMG sensors) or other types of data from other sensors, such as proximity sensors, ToF sensors, sensors of an IMU, capacitive sensors, strain sensors) detected by a wearable device worn by the user and/or other electronic devices in the user's possession (e.g., smartphones, laptops, imaging devices, intermediary devices, and/or other devices described herein).

The input modalities as alluded to above can be varied and are dependent on a user's experience. For example, in an interaction in which a wrist-wearable device is used, a user can provide inputs using in-air or surface-contact gestures that are detected using neuromuscular signal sensors of the wrist-wearable device. In the event that a wrist-wearable device is not used, alternative and entirely interchangeable input modalities can be used instead, such as camera(s) located on the headset or elsewhere to detect in-air or surface-contact gestures or inputs at an intermediary processing device (e.g., through physical input components (e.g., buttons and trackpads)). These different input modalities can be interchanged based on both desired user experiences, portability, and/or a feature set of the product (e.g., a low-cost product may not include hand-tracking cameras).

While the inputs are varied, the resulting outputs stemming from the inputs are also varied. For example, an in-air gesture input detected by a camera of a head-wearable device can cause an output to occur at a head-wearable device or control another electronic device different from the head-wearable device. In another example, an input detected using data from a neuromuscular signal sensor can also cause an output to occur at a head-wearable device or control another electronic device different from the head-wearable device. While only a couple of examples are described above, one skilled in the art would understand that different input modalities are interchangeable along with different output modalities in response to the inputs.

Specific operations described above may occur as a result of specific hardware. The devices described are not limiting and features on these devices can be removed or additional features can be added to these devices. The different devices can include one or more analogous hardware components. For brevity, analogous devices and components are described herein. Any differences in the devices and components are described below in their respective sections.

As described herein, a processor (e.g., a central processing unit (CPU) or microcontroller unit (MCU)), is an electronic component that is responsible for executing instructions and controlling the operation of an electronic device (e.g., a wrist-wearable device, a head-wearable device, a handheld intermediary processing device (HIPD), a smart textile-based garment, or other computer system). There are various types of processors that may be used interchangeably or specifically required by embodiments described herein. For example, a processor may be (i) a general processor designed to perform a wide range of tasks, such as running software applications, managing operating systems, and performing arithmetic and logical operations; (ii) a microcontroller designed for specific tasks such as controlling electronic devices, sensors, and motors; (iii) a graphics processing unit (GPU) designed to accelerate the creation and rendering of images, videos, and animations (e.g., VR animations, such as three-dimensional modeling); (iv) a field-programmable gate array (FPGA) that can be programmed and reconfigured after manufacturing and/or customized to perform specific tasks, such as signal processing, cryptography, and machine learning; or (v) a digital signal processor (DSP) designed to perform mathematical operations on signals such as audio, video, and radio waves. One of skill in the art will understand that one or more processors of one or more electronic devices may be used in various embodiments described herein.

As described herein, controllers are electronic components that manage and coordinate the operation of other components within an electronic device (e.g., controlling inputs, processing data, and/or generating outputs). Examples of controllers can include (i) microcontrollers, including small, low-power controllers that are commonly used in embedded systems and Internet of Things (IoT) devices; (ii) programmable logic controllers (PLCs) that may be configured to be used in industrial automation systems to control and monitor manufacturing processes; (iii) system-on-a-chip (SoC) controllers that integrate multiple components such as processors, memory, I/O interfaces, and other peripherals into a single chip; and/or (iv) DSPs. As described herein, a graphics module is a component or software module that is designed to handle graphical operations and/or processes and can include a hardware module and/or a software module.

As described herein, memory refers to electronic components in a computer or electronic device that store data and instructions for the processor to access and manipulate. The devices described herein can include volatile and non-volatile memory. Examples of memory can include (i) random access memory (RAM), such as DRAM, SRAM, DDR RAM or other random access solid state memory devices, configured to store data and instructions temporarily; (ii) read-only memory (ROM) configured to store data and instructions permanently (e.g., one or more portions of system firmware and/or boot loaders); (iii) flash memory, magnetic disk storage devices, optical disk storage devices, other non-volatile solid state storage devices, which can be configured to store data in electronic devices (e.g., universal serial bus (USB) drives, memory cards, and/or solid-state drives (SSDs)); and (iv) cache memory configured to temporarily store frequently accessed data and instructions. Memory, as described herein, can include structured data (e.g., SQL databases, MongoDB databases, GraphQL data, or JSON data). Other examples of memory can include (i) profile data, including user account data, user settings, and/or other user data stored by the user; (ii) sensor data detected and/or otherwise obtained by one or more sensors; (iii) media content data including stored image data, audio data, documents, and the like; (iv) application data, which can include data collected and/or otherwise obtained and stored during use of an application; and/or (v) any other types of data described herein.

As described herein, a power system of an electronic device is configured to convert incoming electrical power into a form that can be used to operate the device. A power system can include various components, including (i) a power source, which can be an alternating current (AC) adapter or a direct current (DC) adapter power supply; (ii) a charger input that can be configured to use a wired and/or wireless connection (which may be part of a peripheral interface, such as a USB, micro-USB interface, near-field magnetic coupling, magnetic inductive and magnetic resonance charging, and/or radio frequency (RF) charging); (iii) a power-management integrated circuit, configured to distribute power to various components of the device and ensure that the device operates within safe limits (e.g., regulating voltage, controlling current flow, and/or managing heat dissipation); and/or (iv) a battery configured to store power to provide usable power to components of one or more electronic devices.

As described herein, peripheral interfaces are electronic components (e.g., of electronic devices) that allow electronic devices to communicate with other devices or peripherals and can provide a means for input and output of data and signals. Examples of peripheral interfaces can include (i) USB and/or micro-USB interfaces configured for connecting devices to an electronic device; (ii) Bluetooth interfaces configured to allow devices to communicate with each other, including Bluetooth low energy (BLE); (iii) near-field communication (NFC) interfaces configured to be short-range wireless interfaces for operations such as access control; (iv) pogo pins, which may be small, spring-loaded pins configured to provide a charging interface; (v) wireless charging interfaces; (vi) global-positioning system (GPS) interfaces; (vii) Wi-Fi interfaces for providing a connection between a device and a wireless network; and (viii) sensor interfaces.

As described herein, sensors are electronic components (e.g., in and/or otherwise in electronic communication with electronic devices, such as wearable devices) configured to detect physical and environmental changes and generate electrical signals. Examples of sensors can include (i) imaging sensors for collecting imaging data (e.g., including one or more cameras disposed on a respective electronic device, such as a simultaneous localization and mapping (SLAM) camera); (ii) biopotential-signal sensors; (iii) IMUs for detecting, for example, angular rate, force, magnetic field, and/or changes in acceleration; (iv) heart rate sensors for measuring a user's heart rate; (v) peripheral oxygen saturation (SpO₂) sensors for measuring blood oxygen saturation and/or other biometric data of a user; (vi) capacitive sensors for detecting changes in potential at a portion of a user's body (e.g., a sensor-skin interface) and/or the proximity of other devices or objects; (vii) sensors for detecting some inputs (e.g., capacitive and force sensors); and (viii) light sensors (e.g., ToF sensors, infrared light sensors, or visible light sensors), and/or sensors for sensing data from the user or the user's environment. As described herein, biopotential-signal-sensing components are devices used to measure electrical activity within the body (e.g., biopotential-signal sensors). Some types of biopotential-signal sensors include (i) electroencephalography (EEG) sensors configured to measure electrical activity in the brain to diagnose neurological disorders; (ii) electrocardiography (ECG or EKG) sensors configured to measure electrical activity of the heart to diagnose heart problems; (iii) EMG sensors configured to measure the electrical activity of muscles and diagnose neuromuscular disorders; (iv) electrooculography (EOG) sensors configured to measure the electrical activity of eye muscles to detect eye movement and diagnose eye disorders.

As described herein, an application stored in memory of an electronic device (e.g., software) includes instructions stored in the memory. Examples of such applications include (i) games; (ii) word processors; (iii) messaging applications; (iv) media-streaming applications; (v) financial applications; (vi) calendars; (vii) clocks; (viii) web browsers; (ix) social media applications; (x) camera applications; (xi) web-based applications; (xii) health applications; (xiii) AR and MR applications; and/or (xiv) any other applications that can be stored in memory. The applications can operate in conjunction with data and/or one or more components of a device or communicatively coupled devices to perform one or more operations and/or functions.

As described herein, communication interface modules can include hardware and/or software capable of data communications using any of a variety of custom or standard wireless protocols (e.g., IEEE 802.15.4, Wi-Fi, ZigBee, 6LoWPAN, Thread, Z-Wave, Bluetooth Smart, ISA100.11a, WirelessHART, or MiWi), custom or standard wired protocols (e.g., Ethernet or HomePlug), and/or any other suitable communication protocol, including communication protocols not yet developed as of the filing date of this document. A communication interface is a mechanism that enables different systems or devices to exchange information and data with each other, including hardware, software, or a combination of both hardware and software. For example, a communication interface can refer to a physical connector and/or port on a device that enables communication with other devices (e.g., USB, Ethernet, HDMI, or Bluetooth). A communication interface can refer to a software layer that enables different software programs to communicate with each other (e.g., APIs and protocols such as HTTP and TCP/IP).

As described herein, a graphics module is a component or software module that is designed to handle graphical operations and/or processes and can include a hardware module and/or a software module.

As described herein, non-transitory computer-readable storage media are physical devices or storage medium that can be used to store electronic data in a non-transitory form (e.g., such that the data is stored permanently until it is intentionally deleted and/or modified).

### Wrist-Wearable Device with an Electromyography Sensor and a Photoplethysmogram Sensor

Figure 1 illustrates a wrist-wearable device 100 that includes a heart rate monitor (HRM) and an electromyography (EMG) sensor, in accordance with some embodiments. In the depicted example, the wrist-wearable device 100 includes features, structures, and/or configurations that allow for the heart rate monitor and the electromyography sensor to each accurately sense or detect heart rate and electromyography signals. For example, in some embodiments, the wrist-wearable device 100 includes a bottom capsule portion 102 that supports both heart rate monitor and the electromyography functionality. Multiple views of a bottom capsule portion 102 of the wrist-wearable device 100 is shown in an exploded view 104.

In the depicted example, the wrist-wearable device 100 includes a heart rate monitor. In some embodiments, the heart rate monitor is an optical heart rate monitor, such as a photoplethysmogram (PPG) sensor configured to provide information for determining a heart rate of the user. As illustrated in bottom view 106, the bottom capsule portion 102 can include one or more light pipes to facilitate the transmission of light to and/or from the PPG sensor. For example, the bottom view 106 illustrates light pipes illustrated as a plurality of light emitter outlets 110A-110C to transmit light from the PPG sensor to the user and light receiver inlets 112A-112D to transmit light received from the user to the PPG sensor.

In some embodiments, the light pipes forming the light emitter outlets 110A-110C and the light receiver inlets 112A-112D are injection molded as a portion of the bottom capsule portion 102. In some applications, the light pipes are formed from a clear material. Further, in some embodiments, the injection molding of the light pipes may be a first shot of a multi-shot injection molded bottom capsule portion 102.

In the depicted example, the wrist-wearable device 100 further includes an electromyography sensor that receives biopotential information (e.g. electrical signals) of a user for determining a movement of the user. As illustrated in bottom view 106, the bottom capsule portion 102 can include one or more electrode contacts 108A-108F to facilitate transmission of electrical or biopotential signals to and/or from the EMG sensor of the wrist-wearable device.

In some embodiments, the electrode contacts 108A-108F are conductive inserts formed into the bottom capsule portion 102. In some applications, the conductive inserts are stainless steel inserts. In the depicted example, the electrode contacts 108A-108F are inserted as part of the molding process of the bottom capsule portion 102. In some applications, the electrode contacts 108A-108F may be inserted during a second shot of the multi-shot injection molded bottom capsule portion 102. In some embodiments, the first and second shot of the injection molded bottom capsule portion 102 can be molecularly bonded (e.g. plastic to plastic molecular bonding). In some embodiments, the bottom capsule portion 102 can be formed from multiple pieces. Further, in some embodiments, glue wells can seal and/or connect metal components to plastic components of the bottom capsule portion 102.

In some embodiments, a center portion of the bottom capsule portion 102 is raised to enhance skin contact between the light emitter outlets 110A-110C and the light receiver inlets 112A-112D and the user's skin to improve HRM sensing and to enhance contact between the electrode contacts 108A-108F and the user's skin to reduce skin to electrode impedance and/or potential contact loss. In some embodiments, the center portion of the bottom capsule portion 102 is a square (e.g. a 25 mm by 25 mm portion) that is raised relative to the other portions of the bottom capsule portion 102.

In addition, the bottom view 106 also shows two charging contacts 114A and 114B which can couple with a charger to charge a battery of the wrist-wearable device. While two charging contacts are shown, additional contacts can be used for charging purposes and/or data transfer.

Figure 1 shows an interior view 116 of the bottom capsule portion 102 which shows a printed circuit board (PCB) 118 that is configured to control the EMG sensor, the PPG sensor, and optionally the charging of a battery. In some embodiments, the components of the EMG sensor and the PPG sensor are mounted using surface mount technology (SMT) to reduce the overall dimensions of the PCB. In some embodiments, the PCB 118 can include one or more spring contacts to facilitate an electrical connection between the PCB 118 and the electrode contacts 108A-108F. Further, the PCB 118 can include one or more analog front ends (AFEs) for use with the EMG sensors. In some applications, the PCB 118 can include integrated shielding. In some embodiments, more functions or fewer functions can be controlled by the PCB 118. For example, a PPG emitter module can be attached directly to the PCB 118 via SMT.

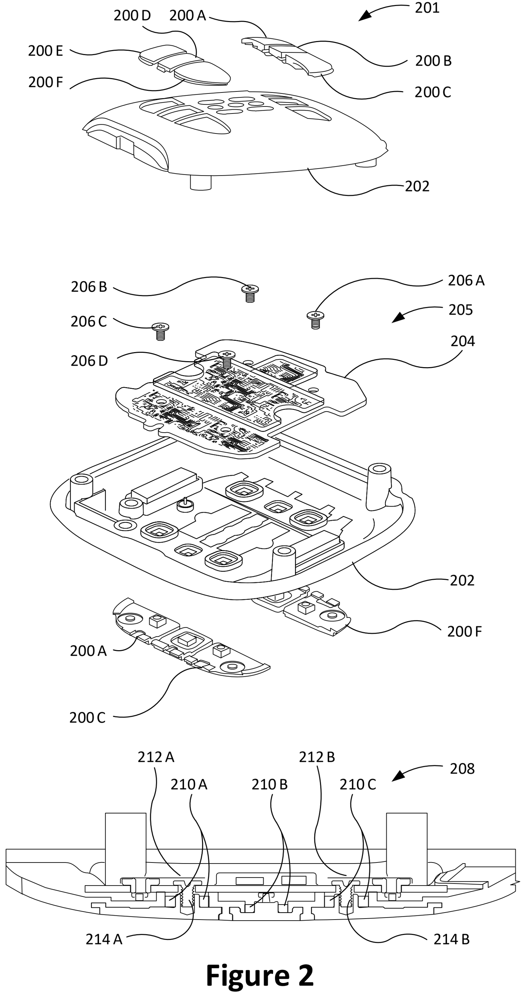

Figure 1 shows a cutaway view 120 of the bottom capsule portion 102, which shows how the EMG sensor components, PPG components, and PCB are packaged within the bottom capsule portion 102. Further details of the components are described in reference to at least Figures 2-4. The cutaway view shows a PPG module 122, an optical sealing gasket 124, and optical lens 126. In the depicted example, the bottom capsule portion 102 can be sealed against dust and/or water intrusion.

Figure 1 also shows a chart 128 that indicates that PPG data 130 and EMG data 132 can be concurrently recorded without interfering with each other. The chart also further indicates that the data recorded is sufficiently accurate and is not degraded due to the data recording components for the EMG sensor and PPG sensor being within close proximity to each other.

Figure 2 illustrates the integration of the electrodes 200A-200F of an EMG sensor with a bottom capsule portion 202 of a wrist-wearable device (e.g., wrist-wearable device 100 shown in Figure 1), in accordance with some embodiments. Figure 2 shows a first view 201, which shows the overall shape of the electrodes 200A-200F and the positioning of the electrodes 200A-200F relative to the bottom capsule portion 202. In the depicted example, the conductive electrodes 200A-200F are integrated with the plastic enclosure of the bottom capsule portion 202 to provide a continuous skin-facing surface to facilitate EMG waveform detection by providing a direct electrical connection from the users skin to components within the wrist-wearable device 100, such as the EMG sensor. In some embodiments, the electrodes 200A-200F provide additional structural integrity to the bottom capsule portion 202.

As described herein, the bottom capsule portion 202 can be produced using two shots of material so the electrodes 200A-200F can be partially encapsulated within the bottom capsule portion, i.e., the electrodes 200A-200F are overmolded within and permanently fixed to the bottom capsule portion 202. In some embodiments, the electrodes can be press fit into the bottom capsule portion without needing to be overmolded. In some embodiments, the electrodes can be secured via fasteners. In some embodiments, the electrodes 200A-200F can be formed via a metal injection molding process, a computer numeric control machining process, a forging process, a stamping process, and/or a casting process. Advantageously, embodiments of the wearable device can include features to avoid cracking between the interface of the electrodes 200A-200F and the bottom capsule portion 202. In some applications, the bottom capsule portion 202 can be formed form polycarbonate and/or polybutylene terephthalate and may have a thickness of 0.40mm or thicker. Further, in some applications, the electrodes 200A-200F can include radiused corners and/or edges to further minimize cracking.

Figure 2 shows an example exploded view 205 which shows how the PCB 204 is mounted within the bottom capsule portion 202. As shown in the illustrative exploded view 205, the PCB 204 is secured within the bottom capsule portion 202 via fasteners 206A-206D (e.g., threaded screws) which are configured to couple with threaded inserts on the interior surfaces of some of the electrodes 200A, 200C, 200D (occluded), and 200F. By having the electrodes provide a secondary function of securing the PCB the overall weight and dimensions can be reduced as compared to a wrist-wearable device that does not use a multi-use electrode. In some embodiments, the screw transmits biometric signals to other components of the EMG sensor located on the PCB. In some embodiments, the screw does not transmit biometric signals. Further, in some embodiments, the electrodes 200A-200F are mechanically shielded from power line interference (PLI). In some applications, PLI shielding is attached to the electrodes 200A-200F. Advantageously, PLI shielding can improve the signal to noise ratio of the EMG sensor.

Figure 2 also shows a cutaway view 208 of a side of electrodes and illustrates sealing gaskets 210A-210C and 210D-210F (not illustrated in this view). In the depicted example, a flange of the bottom capsule portion 202 can seal the electrodes 200A-200F and the bottom capsule portion 202 from water and dust ingress. In some applications, sealing gaskets 210A-210F correspond to each of the electrodes 200A-200F and prevent moisture and debris ingress into the internal components of the wrist-wearable device, such as the conductive pathways between the electrodes 200A-200F and the EMG disposed on the PCB. In some embodiments, the gasket is a foam or other type of compressible gasket. In some embodiments, the bottom capsule portion 202 includes a sealing glue or adhesive, such as an epoxy configured to create a seal with the electrodes 200A-200F. Cutaway view 208 also shows sheet metal pieces 212A and 212B that cover the screws 214A and 214B. In some embodiments, conductive surfaces in proximity to the sealing portions can facilitate an electrical connection between the EMG disposed on the PCB and the electrodes 200A-200F. Advantageously, the sealed design of the bottom capsule portion 202 can resist drops, chemical exposure, and user wear and tear.

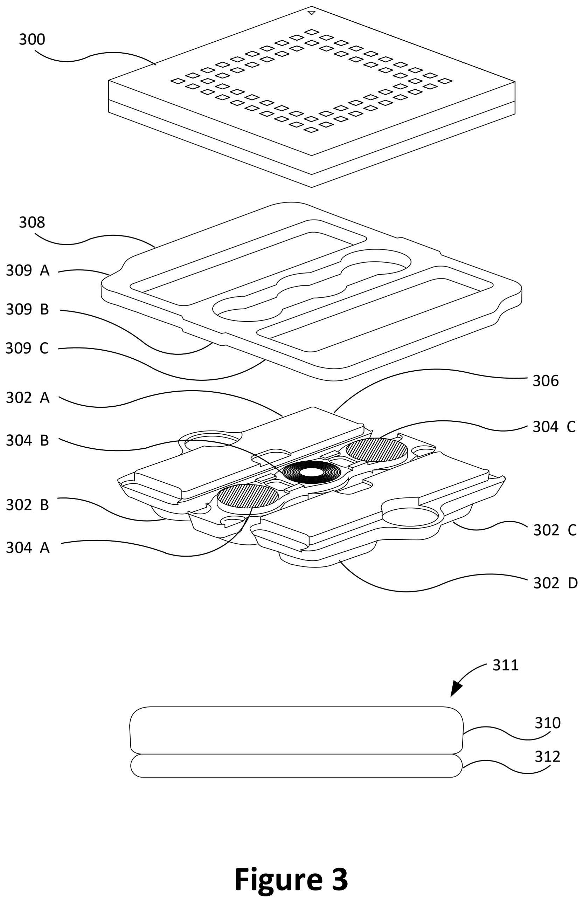

Figure 3 illustrates a PPG assembly that uses an optical sealing gasket to reduce light bleed between the emitters and the receivers, in accordance with some embodiments. Figure 3 shows a PPG module 300 that is attached, via SMT, to the PCB 204 shown in Figure 2. The PPG module includes an emitter(s) and receiver(s) which in this view are occluded but correspond to inlets 302A-302D and outlets 304A-304C located on an optical lens 306. To ensure light from the emitters does not reach the receiver prior to the light reaching the skin of a user, an optical sealing gasket 308 is placed between the PPG module and the optical lens 306 to minimize cross talk. The optical sealing gasket 308 can include cutouts for the inlets 302A-302D and the outlets 304A-304C to isolate the inlets from the outlets. In some embodiments, the lower portion of the optical lens 306 opposite to the optical sealing gasket can be surrounded by an overmolded material (e.g., plastic) to limit light bleed and cross talk between the emitters and receivers, such that the overmolded plastic operates in a similar manner to the optical sealing gasket 308.

Figure 3 also shows a cutaway view 311 which illustrates that the optical sealing gasket is comprised of at least two layers, which can include a low compression force, higher density foam portion 310 and a light-blocking pressure-sensitive adhesive (PSA) portion 312. Advantageously, the highly flexible and compressible optical sealing gasket 308 facilitates light sealing over a wide range of tolerances, allowing for the integration of both an EMG sensor and a PPG sensor in a common minimized form factor. Further, the optical sealing gasket 308 allows for integration of EMG sensors and PPG sensors in a common architecture, by providing a low force compressibility, good light-blocking characteristics, and minimal thickness to fit within the wrist-wearable device. Having a high compressibility allows for greater tolerances and also reduces the stress on sensitive components within the wrist wearable device (e.g., the PPG module, the optical lens, and the PCB). Advantageously, the use of optical sealing gasket 308 can allow for optical sealing without requiring the compressive force and/or direct attachment to plastic substrates required by certain conventional pressure sensitive adhesives.

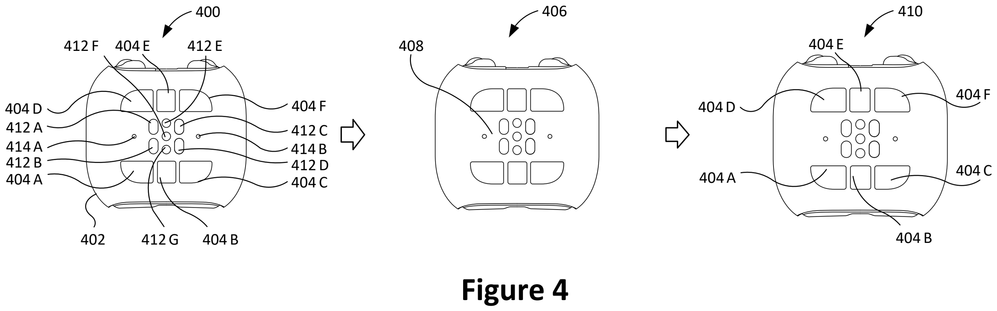

Figure 4 illustrates a method in which a hard coat is applied to a surface of the wrist-wearable device while ensuring skin contact portions of the electrodes are not impeded, in accordance with some embodiments. Advantageously, a hard coat can protect the bottom cover plastic from abrasion and/or chemical degradation. As described herein, the hard coat can be removed from the electrodes to allow for detection of the EMG waveform.

As shown in a first step 400, the bottom cover 402 with the electrodes 404A-404F installed is provided for the hard coat process. In the second step 406 a hard coat 408 is applied to all surfaces on the bottom cover 402 (e.g., applied to just the skin-facing portion of the bottom cover 402 or all of it depending on the hard coat selected). As described herein, the hard coat 408 can protect exposed plastic surfaces from abrasions (e.g. scratches and dents from drops or engaging with a charging mechanism) and/or chemical degradation (e.g. from perfume, sunscreen and/or sweat).

In a third step 410, the hard coat is removed from the electrodes 404A-404F to allow for electrical contact with the user's skin. In some embodiments, the hard coat is removed via laser ablation. In some embodiments, a CCD camera that captures data indicating the edges of the electrodes 404A-404F relative to the other portions of the bottom cover 402 to guide the output of the laser to only ablate the electrodes 404A-404F to remove the hard coat. In some embodiments, the hard coat covers the PPG light inlets and outlets 412A-412G, and the charging contacts 414A-414B. The hard coat does not degrade the functionality of these components as compared to non-hard-coated components. Advantageously, removing the hard coat from desired areas such as electrodes 404A-404F via laser ablation can provide improved cosmetic results compared to masking portions of the bottom cover 402, which may result in poor cosmetic results. Further, removing the hard coat via laser ablation allows the electrodes to be molded directly into the bottom cover. In some embodiments, the hard coat is tinted to react more readily to energy from the laser, which can speed up the ablating process as compared to a non-tinted hard coat. In some embodiments, the hard coat is a diamond-like coating that can vary in color/tinting, thickness, and/or opacity. In some embodiments, the electrodes 404A-404F can be coated with a conductive diamond-like coating.

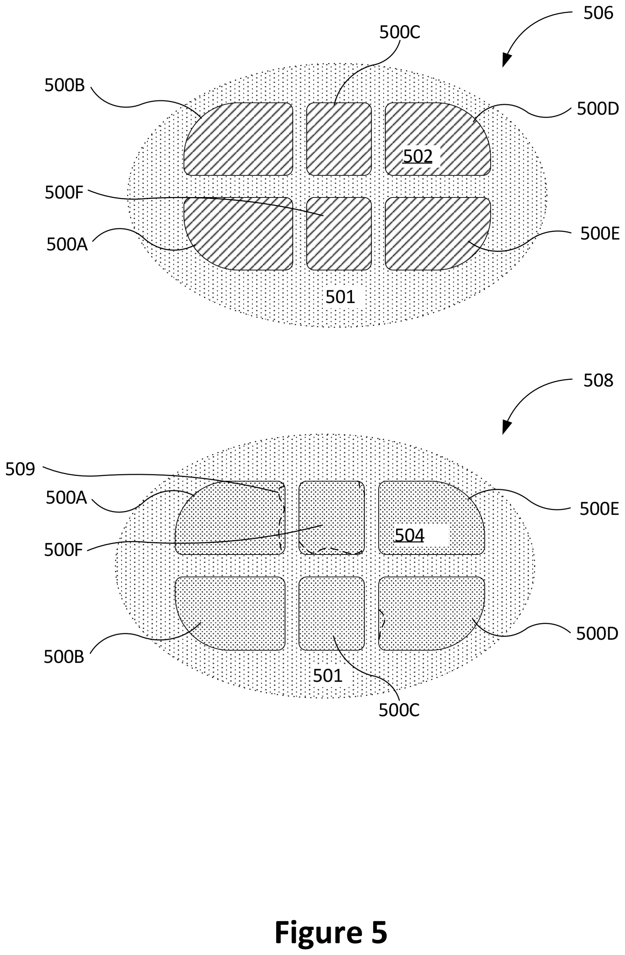

Figure 5 illustrates a method of limiting plastic overflow when overmolding over the electrodes in the bottom cover of a wrist-wearable device, in accordance with some embodiments. As described herein, insert molding of the electrodes 500A-500F into the bottom housing 506 can provide a seamless appearance. In some applications, to limit overflow when overmolding the electrodes 500A-500F into the bottom housing 506, a heavy texture, such as a VDI-15 surface finish 502, is applied to the surface of the electrodes 500A-500F. In the depicted example, the use of a heavy texture, such as the VDI-15 surface finish 502 can limit plastic overflow of the overmolded material 501 during an injection molding process, allowing for improved control of the flow of the plastic surrounding the electrodes 500A-500F.

Advantageously, the use a heavier texture, such as a VDI-15 surface finish 502 can allow for improved plastic overflow control compared to an electrode with lighter texture, such as a VDI-5 surface finish 504, which is shown in the second example 508 to illustrate the visual differences (e.g., underfill and overfill 509 of the overmolded material 501 around the perimeters of the electrodes 500A-500F). In some embodiments, overflow can also be controlled by keeping the edges of the cosmetic surface sharp. In some embodiments, viscosity of the plastic resin during injection molding can also be used to control overflow consistency.

Numerous additional details that are meant to augment the descriptions provided in reference to Figures 1 to Figure 5 are provided below.

(A1) In accordance with some embodiments, a wrist-wearable device comprises a capsule that includes: an electromyography (EMG) sensor configured to provide biopotential information of a user for determining a movement of the user, and a photoplethysmogram (PPG) sensor configured to provide information for determining a heart rate of the user. For example, Figure 1 shows a wrist-wearable device 100 that includes a heart rate monitor (HRM), e.g., a PPG based heart rate monitor, and an electromyography (EMG) sensor. The biopotential information and information for determining the heart rate of the user is received simultaneously, and sensing of the biopotential information does not alter the information for determining heart rate of the user, and sensing of the information for determining heart rate of the user does not alter the biopotential information. For example, Figure 1 shows a chart 128 that illustrates the data being received from the PPG sensor is not degraded while the data from the EMG sensor is being received, and vice versa.

(A2) In some embodiments of A1, electrodes of the EMG sensor are overmolded into the capsule and are a structural component of the capsule. For example, cutaway view 208 in Figure 2 shows how the electrodes of the EMG sensor are overmolded and integrally formed with the bottom capsule portion 202. The electrodes are load bearing and are designed to be a structural component of the bottom capsule portion, thereby reducing the need for extra material for increasing the rigidity of the wrist-wearable device.

(A3) In some embodiments of A2, at least one of the electrodes includes an interface for receiving a fastener, wherein the fastener is configured to secure a PCB within the capsule. For example, Figure 2 illustrates that PCB 204 is secured within the bottom capsule portion 202 via fasteners 206A-206D (e.g., threaded screws) which are configured to couple with threaded inserts on the interior surfaces of some of the electrodes 200A, 200C, 200D, and 200F.

(A4) In some embodiments of any one of A1-A3, at least some of the components of the EMG sensor and at least some of the components of the PPG sensor are integrated into a common printed circuit board (PCB). For example, Figure 2 shows a PCB 204 that includes one or more analog front ends (AFEs) for the electrodes 200A-200F and a PPG module attached via SMT to the PCB 204 (also shown in Figure 1 as PPG module 122 being attached to PCB 118).

(A5) In some embodiments of A4, the PCB includes one or more electrical components configured to control the EMG sensor (e.g., analog front ends (AFEs) and shielding) and PPG sensor (e.g., a PPG module that includes an emitter (LEDs) and a receiver (e.g., photodiodes)). For example, Figure 2 shows a PCB 204 that includes one or more analog front ends (AFEs) for the electrodes 200A-200F and a PPG module attached via SMT to the PCB 204 (also shown in Figure 1 as PPG module 122 being attached to PCB 118). In some embodiments, other additional components can be integrated into the PCB, such as charging components configured to charge a battery of the wrist-wearable device, a speaker, a processor(s) for processing functions of the wrist-wearable device, a communication component (e.g., Bluetooth, WiFi, etc.), microphone, haptic feedback generator, etc.

(A6) In some embodiments of A4, the PCB further includes one or more components for facilitating charging of a battery of the wrist-wearable device. For example, Figure 1 shows two charging contacts 114A and 114B which can couple with a charger to charge a battery of the wrist-wearable device.

(A7) In some embodiments of A3, the fastener at least partially secures an optical sealing gasket between an interior surface of the capsule and the PCB, and the optical sealing gasket is configured to seal light from bleeding from the emitters to the receivers of the PPG sensor before exiting the capsule. For example, Figure 2 shows a PCB 204 is secured within the bottom capsule portion 202 via fasteners 206A-206D (e.g., threaded screws) which are configured to couple with threaded inserts on the interior surfaces of some of the electrodes 200A, 200C, 200D, and 200F. In between the PCB 204 and the bottom capsule portion 202 an optical sealing gasket 308 (shown in Figure 3) is placed. Figure 1 also shows in cutaway view 120 of an optical sealing gasket 124 placed between a PPG module 122 and optical lens 126. Figure 1 also illustrates the highly compressible nature of the optical sealing gasket, which contours and compresses to the different surfaces of the optical lens and the PPG module within the capsule.

(A8) In some embodiments of A3, a power line interference (PLI) fastener shield is placed around the fastener to minimize power line interference of the EMG sensor. For example, Figure 2 shows that cutaway view 208 also shows sheet metal pieces 212A and 212B that cover the screws 214A and 214B.

(A9) In some embodiments of any of A1-A8, a surface of the EMG sensor is coplanar with a surface of the PPG sensor. For example, Figure 1 shows the optical lens being coplanar with the electrodes of the EMG sensor in the cutaway view 120.

(A10) In some embodiments of any of A1-A9, the electrodes of the EMG sensor and the outlet of emitters and receivers of the PPG sensor are located on a protrusion of the capsule, wherein the protrusion is configured to maintain contact with the skin of a user while donned. For example, Figure 1 shows in cutaway view 120 the optical lens and the electrodes of the EMG sensor are affixed to a protrusion 127.

(A11) In some embodiments of A10, the protrusion includes one or more charging contacts for providing power to a battery of the capsule. For example, Figure 1 shows charging contacts 114A and 114B placed on the protrusion 127.

(A12) In some embodiments of any of A1-A11, the PPG sensor is comprised of a PPG module that includes an emitter and a receiver, a sealing gasket that is configured to be coupled to the PPG module, and an optical lens configured to transfer emitted light of the emitter to the skin of the user and to transfer reflected light to the receiver. For example, Figure 3 illustrates a PPG module 300, an optical sealing gasket 308, and an optical lens 306.

(A13) In some embodiments of A12, the capsule includes one or more pass-throughs that allow for portions of the optical lens to pass through the capsule, and the portions of the optical lens are configured to be in contact with the skin of a user. As shown in Figure 3, the optical sealing gasket includes cutouts 309A, 309B, and 309C that are configured to allow for portions of the optical lens 306 to pass through.

(A14) In some embodiments of any one of A1-A13, an optical sealing gasket of the PPG sensor is a multilayer gasket. For example, Figure 3 illustrates a cutaway view 311 that shows that the optical sealing gasket is comprised of at least two layers.

(A15) In some embodiments of A14, the multilayer gasket is comprised of a low compression force, higher density foam portion and a light blocking pressure-sensitive adhesive (PSA) portion. Figure 3 illustrates in cutaway view 311 the optical sealing gasket is comprised of at least two layers, which can include a low compression force, higher density foam portion 310 and a light blocking pressure-sensitive adhesive (PSA) portion 312.

(A16) In some embodiments of any one of A1-A15, electrodes of the EMG sensor are sealed from a cavity of the capsule by an ingress seal. For example, Figure 2 also shows a cutaway view 208 of a side of electrodes and illustrates sealing gaskets 210A-210C and 210D-210F (not illustrated in this view).

(A17) In some embodiments of any one of A1-A16, a hard coat is applied to the capsule, and the hard coat does not cover electrodes of the EMG sensor. For example, Figure 4 illustrates in step 410 that a hard coat 408 is applied to the bottom cover 402 and is removed from the electrodes 404A-404F.

(B1) In accordance with some embodiments, a gasket of a wrist-wearable device, comprising a light-noise reducing cutout configured isolate light of an emitter of a photoplethysmogram (PPG) sensor from a receiver of the PPG sensor while the light is being transmitted within the capsule, such that the PPG sensor can record information for determining the heart rate of a user. The gasket has a shape that is configured to accommodate one or more components of an electromyography (EMG) sensor placed around a perimeter of the gasket, and the EMG sensor is configured to provide biopotential information of the user for determining a movement of the user.

(C1) In accordance with some embodiments, an electronic device comprises a wrist-wearable device comprising a capsule portion and a band portion. The capsule portion comprises a bottom cover including a protrusion on an external portion of the capsule portion configured to interface with a portion of the user's skin, wherein the bottom portion includes one or more electrode contacts. The capsule portion also comprises a sensor board coupled to an internal portion of the capsule portion and electrically coupled to the one or more electrode contacts via one or more screws, and an optical sealing gasket including at least two layers configured to be flexible and compressible and configured to seal light leakage from an emitter from an optical sensor. In some embodiments, a hard coat is applied to the bottom cover excluding the one or more electrode contacts configured to protect the capsule portion from chemicals (e.g., corrosion caused by perfume, sunscreen, skin oils, sweat, etc.).

(D1) In accordance with some embodiments, the wrist-wearable device of any one of A1-A19, wherein the one or more programs of the wrist-wearable device include instructions for interacting with an extended-reality environment.

(E1) In accordance with some embodiments, the wrist-wearable device of any one of A1-A19, wherein the wrist-wearable device includes a non-transitory, computer-readable storage medium including executable instructions that, when executed by one or more processors, cause the one or more processors to perform or cause performance operations for interacting with an extended-reality environment.

(F1) In accordance with some embodiments, the wrist-wearable device of any one of A1-A19, wherein the wrist-wearable device includes a means for performing or causing performance of operations for interacting with an extended-reality environment.

(G1) In accordance with some embodiments, a system comprising a wrist-wearable device configured in accordance with any one of A1-A19 and a pair of glasses communicatively coupled to the wrist-wearable device.

An example wrist-wearable device comprises a capsule that includes an electromyography (EMG) sensor configured to provide biopotential information of a user for determining a movement of the user, and a photoplethysmogram (PPG) sensor configured to provide information for determining the heart rate of the user. The biopotential information and information for determining the heart rate of the user is received simultaneously. Additionally, sensing of the biopotential information does not alter the information for determining the heart rate of the user, and sensing of the information for determining the heart rate of the user does not alter the biopotential information.

### Example Extended-Reality Systems

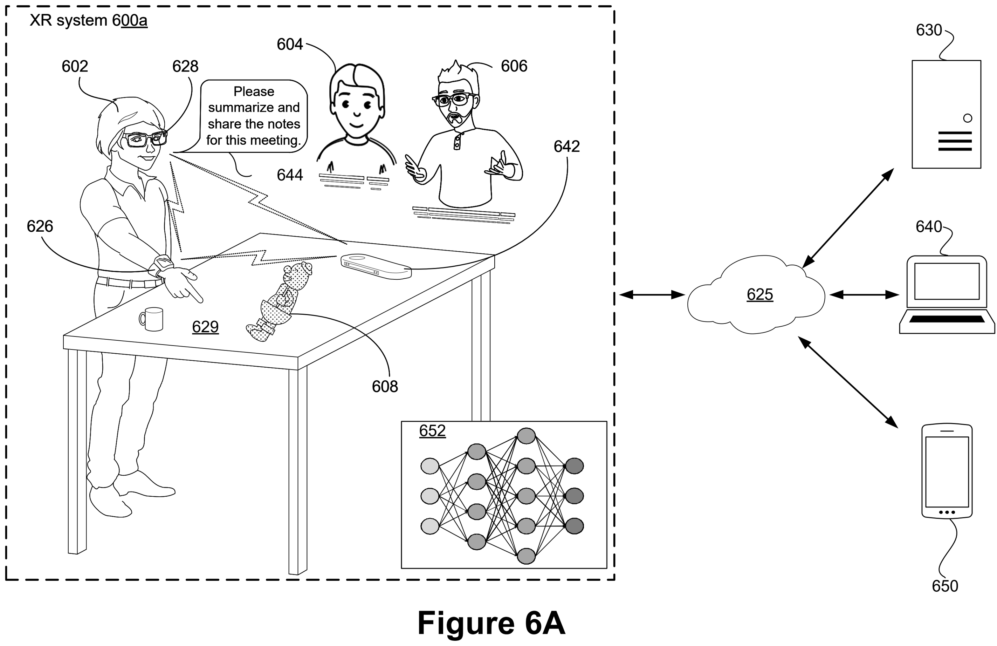

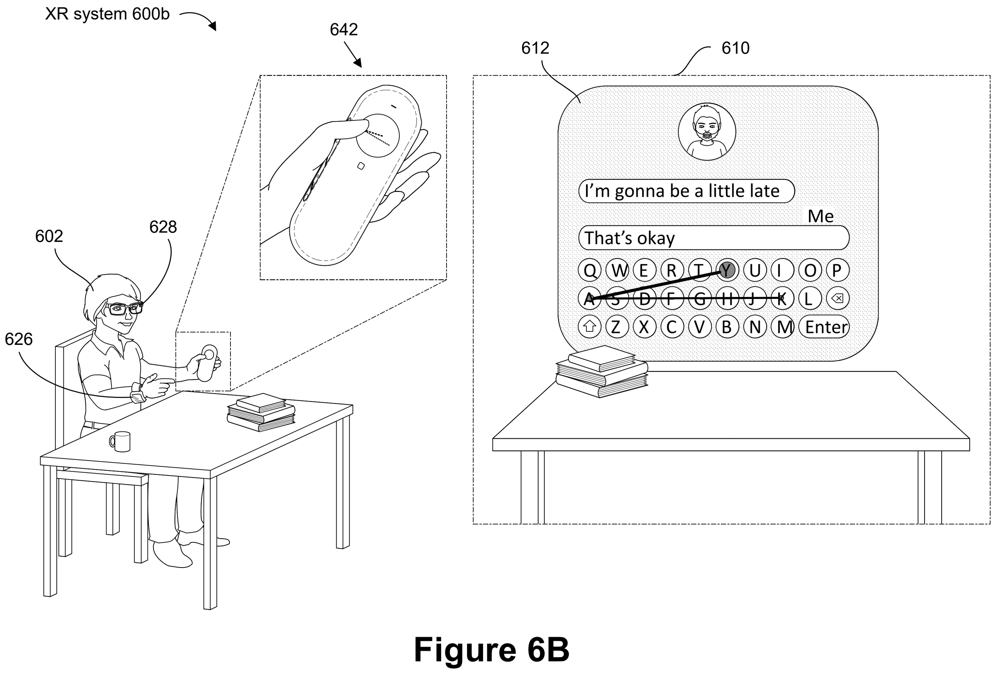

Figures 6A, 6B, 6C-1, and 6C-2, illustrate example XR systems that include AR and MR systems in accordance with some embodiments. Figure 6A shows a first XR system 600a and first example user interactions using a wrist-wearable device 626, a head-wearable device (e.g., AR device 628), and/or a HIPD 642. Figure 6B shows a second XR system 600b and second example user interactions using a wrist-wearable device 626, AR device 628, and/or an HIPD 642. Figures 6C-1, and 6C-2 show a third MR system 600c and third example user interactions using a wrist-wearable device 626, a head-wearable device (e.g., an MR device such as a VR device), and/or an HIPD 642. As the skilled artisan will appreciate upon reading the descriptions provided herein, the above-example AR and MR systems (described in detail below) can perform various functions and/or operations.

The wrist-wearable device 626, the head-wearable devices, and/or the HIPD 642 can communicatively couple via a network 625 (e.g., cellular, near field, Wi-Fi, personal area network, wireless LAN). Additionally, the wrist-wearable device 626, the head-wearable device, and/or the HIPD 642 can also communicatively couple with one or more servers 630, computers 640 (e.g., laptops, computers), mobile devices 650 (e.g., smartphones, tablets), and/or other electronic devices via the network 625 (e.g., cellular, near field, Wi-Fi, personal area network, wireless LAN). Similarly, a smart textile-based garment, when used, can also communicatively couple with the wrist-wearable device 626, the head-wearable device(s), the HIPD 642, the one or more servers 630, the computers 640, the mobile devices 650, and/or other electronic devices via the network 625 to provide inputs.

Turning to Figure 6A, a user 602 is shown wearing the wrist-wearable device 626 and the AR device 628 and having the HIPD 642 on their desk. The wrist-wearable device 626, the AR device 628, and the HIPD 642 facilitate user interaction with an AR environment. In particular, as shown by the first AR system 600a, the wrist-wearable device 626, the AR device 628, and/or the HIPD 642 cause presentation of one or more avatars 604, digital representations of contacts 606, and virtual objects 608. As discussed below, the user 602 can interact with the one or more avatars 604, digital representations of the contacts 606, and virtual objects 608 via the wrist-wearable device 626, the AR device 628, and/or the HIPD 642. In addition, the user 602 is also able to directly view physical objects in the environment, such as a physical table 629, through transparent lens(es) and waveguide(s) of the AR device 628. Alternatively, an MR device could be used in place of the AR device 628 and a similar user experience can take place, but the user would not be directly viewing physical objects in the environment, such as table 629, and would instead be presented with a virtual reconstruction of the table 629 produced from one or more sensors of the MR device (e.g., an outward facing camera capable of recording the surrounding environment).

The user 602 can use any of the wrist-wearable device 626, the AR device 628 (e.g., through physical inputs at the AR device and/or built-in motion tracking of a user's extremities), a smart-textile garment, externally mounted extremity tracking device, the HIPD 642 to provide user inputs, etc. For example, the user 602 can perform one or more hand gestures that are detected by the wrist-wearable device 626 (e.g., using one or more EMG sensors and/or IMUs built into the wrist-wearable device) and/or AR device 628 (e.g., using one or more image sensors or cameras) to provide a user input. Alternatively, or additionally, the user 602 can provide a user input via one or more touch surfaces of the wrist-wearable device 626, the AR device 628, and/or the HIPD 642, and/or voice commands captured by a microphone of the wrist-wearable device 626, the AR device 628, and/or the HIPD 642. The wrist-wearable device 626, the AR device 628, and/or the HIPD 642 include an artificially intelligent digital assistant to help the user in providing a user input (e.g., completing a sequence of operations, suggesting different operations or commands, providing reminders, confirming a command). For example, the digital assistant can be invoked through an input occurring at the AR device 628 (e.g., via an input at a temple arm of the AR device 628). In some embodiments, the user 602 can provide a user input via one or more facial gestures and/or facial expressions. For example, cameras of the wrist-wearable device 626, the AR device 628, and/or the HIPD 642 can track the user 602's eyes for navigating a user interface.

The wrist-wearable device 626, the AR device 628, and/or the HIPD 642 can operate alone or in conjunction to allow the user 602 to interact with the AR environment. In some embodiments, the HIPD 642 is configured to operate as a central hub or control center for the wrist-wearable device 626, the AR device 628, and/or another communicatively coupled device. For example, the user 602 can provide an input to interact with the AR environment at any of the wrist-wearable device 626, the AR device 628, and/or the HIPD 642, and the HIPD 642 can identify one or more back-end and front-end tasks to cause the performance of the requested interaction and distribute instructions to cause the performance of the one or more back-end and front-end tasks at the wrist-wearable device 626, the AR device 628, and/or the HIPD 642. In some embodiments, a back-end task is a background-processing task that is not perceptible by the user (e.g., rendering content, decompression, compression, application-specific operations), and a front-end task is a user-facing task that is perceptible to the user (e.g., presenting information to the user, providing feedback to the user). The HIPD 642 can perform the back-end tasks and provide the wrist-wearable device 626 and/or the AR device 628 operational data corresponding to the performed back-end tasks such that the wrist-wearable device 626 and/or the AR device 628 can perform the front-end tasks. In this way, the HIPD 642, which has more computational resources and greater thermal headroom than the wrist-wearable device 626 and/or the AR device 628, performs computationally intensive tasks and reduces the computer resource utilization and/or power usage of the wrist-wearable device 626 and/or the AR device 628.

In the example shown by the first AR system 600a, the HIPD 642 identifies one or more back-end tasks and front-end tasks associated with a user request to initiate an AR video call with one or more other users (represented by the avatar 604 and the digital representation of the contact 606) and distributes instructions to cause the performance of the one or more back-end tasks and front-end tasks. In particular, the HIPD 642 performs back-end tasks for processing and/or rendering image data (and other data) associated with the AR video call and provides operational data associated with the performed back-end tasks to the AR device 628 such that the AR device 628 performs front-end tasks for presenting the AR video call (e.g., presenting the avatar 604 and the digital representation of the contact 606).

In some embodiments, the HIPD 642 can operate as a focal or anchor point for causing the presentation of information. This allows the user 602 to be generally aware of where information is presented. For example, as shown in the first AR system 600a, the avatar 604 and the digital representation of the contact 606 are presented above the HIPD 642. In particular, the HIPD 642 and the AR device 628 operate in conjunction to determine a location for presenting the avatar 604 and the digital representation of the contact 606. In some embodiments, information can be presented within a predetermined distance from the HIPD 642 (e.g., within five meters). For example, as shown in the first AR system 600a, virtual object 608 is presented on the desk some distance from the HIPD 642. Similar to the above example, the HIPD 642 and the AR device 628 can operate in conjunction to determine a location for presenting the virtual object 608. Alternatively, in some embodiments, presentation of information is not bound by the HIPD 642. More specifically, the avatar 604, the digital representation of the contact 606, and the virtual object 608 do not have to be presented within a predetermined distance of the HIPD 642. While an AR device 628 is described working with an HIPD, an MR headset can be interacted with in the same way as the AR device 628.

User inputs provided at the wrist-wearable device 626, the AR device 628, and/or the HIPD 642 are coordinated such that the user can use any device to initiate, continue, and/or complete an operation. For example, the user 602 can provide a user input to the AR device 628 to cause the AR device 628 to present the virtual object 608 and, while the virtual object 608 is presented by the AR device 628, the user 602 can provide one or more hand gestures via the wrist-wearable device 626 to interact and/or manipulate the virtual object 608. While an AR device 628 is described working with a wrist-wearable device 626, an MR headset can be interacted with in the same way as the AR device 628.

### Integration of Artificial Intelligence with XR Systems

Figure 6A illustrates an interaction in which an artificially intelligent virtual assistant can assist in requests made by a user 602. The AI virtual assistant can be used to complete open-ended requests made through natural language inputs by a user 602. For example, in Figure 6A the user 602 makes an audible request 644 to summarize the conversation and then share the summarized conversation with others in the meeting. In addition, the AI virtual assistant is configured to use sensors of the XR system (e.g., cameras of an XR headset, microphones, and various other sensors of any of the devices in the system) to provide contextual prompts to the user for initiating tasks.

Figure 6A also illustrates an example neural network 652 used in Artificial Intelligence applications. Uses of Artificial Intelligence (AI) are varied and encompass many different aspects of the devices and systems described herein. AI capabilities cover a diverse range of applications and deepen interactions between the user 602 and user devices (e.g., the AR device 628, an MR device 632, the HIPD 642, the wrist-wearable device 626). The AI discussed herein can be derived using many different training techniques. While the primary AI model example discussed herein is a neural network, other AI models can be used. Non-limiting examples of AI models include artificial neural networks (ANNs), deep neural networks (DNNs), convolution neural networks (CNNs), recurrent neural networks (RNNs), large language models (LLMs), long short-term memory networks, transformer models, decision trees, random forests, support vector machines, k-nearest neighbors, genetic algorithms, Markov models, Bayesian networks, fuzzy logic systems, and deep reinforcement learnings, etc. The AI models can be implemented at one or more of the user devices, and/or any other devices described herein. For devices and systems herein that employ multiple AI models, different models can be used depending on the task. For example, for a natural-language artificially intelligent virtual assistant, an LLM can be used and for the object detection of a physical environment, a DNN can be used instead.

In another example, an AI virtual assistant can include many different AI models and based on the user's request, multiple AI models may be employed (concurrently, sequentially or a combination thereof). For example, an LLM-based AI model can provide instructions for helping a user follow a recipe and the instructions can be based in part on another AI model that is derived from an ANN, a DNN, an RNN, etc. that is capable of discerning what part of the recipe the user is on (e.g., object and scene detection).

As AI training models evolve, the operations and experiences described herein could potentially be performed with different models other than those listed above, and a person skilled in the art would understand that the list above is non-limiting.

A user 602 can interact with an AI model through natural language inputs captured by a voice sensor, text inputs, or any other input modality that accepts natural language and/or a corresponding voice sensor module. In another instance, input is provided by tracking the eye gaze of a user 602 via a gaze tracker module. Additionally, the AI model can also receive inputs beyond those supplied by a user 602. For example, the AI can generate its response further based on environmental inputs (e.g., temperature data, image data, video data, ambient light data, audio data, GPS location data, inertial measurement (i.e., user motion) data, pattern recognition data, magnetometer data, depth data, pressure data, force data, neuromuscular data, heart rate data, temperature data, sleep data) captured in response to a user request by various types of sensors and/or their corresponding sensor modules. The sensors' data can be retrieved entirely from a single device (e.g., AR device 628) or from multiple devices that are in communication with each other (e.g., a system that includes at least two of an AR device 628, an MR device 632, the HIPD 642, the wrist-wearable device 626, etc.). The AI model can also access additional information (e.g., one or more servers 630, the computers 640, the mobile devices 650, and/or other electronic devices) via a network 625.

A non-limiting list of AI-enhanced functions includes but is not limited to image recognition, speech recognition (e.g., automatic speech recognition), text recognition (e.g., scene text recognition), pattern recognition, natural language processing and understanding, classification, regression, clustering, anomaly detection, sequence generation, content generation, and optimization. In some embodiments, AI-enhanced functions are fully or partially executed on cloud-computing platforms communicatively coupled to the user devices (e.g., the AR device 628, an MR device 632, the HIPD 642, the wrist-wearable device 626) via the one or more networks. The cloud-computing platforms provide scalable computing resources, distributed computing, managed AI services, interference acceleration, pre-trained models, APIs and/or other resources to support comprehensive computations required by the AI-enhanced function.

Example outputs stemming from the use of an AI model can include natural language responses, mathematical calculations, charts displaying information, audio, images, videos, texts, summaries of meetings, predictive operations based on environmental factors, classifications, pattern recognitions, recommendations, assessments, or other operations. In some embodiments, the generated outputs are stored on local memories of the user devices (e.g., the AR device 628, an MR device 632, the HIPD 642, the wrist-wearable device 626), storage options of the external devices (servers, computers, mobile devices, etc.), and/or storage options of the cloud-computing platforms.

The AI-based outputs can be presented across different modalities (e.g., audio-based, visual-based, haptic-based, and any combination thereof) and across different devices of the XR system described herein. Some visual-based outputs can include the displaying of information on XR augments of an XR headset, user interfaces displayed at a wrist-wearable device, laptop device, mobile device, etc. On devices with or without displays (e.g., HIPD 642), haptic feedback can provide information to the user 602. An AI model can also use the inputs described above to determine the appropriate modality and device(s) to present content to the user (e.g., a user walking on a busy road can be presented with an audio output instead of a visual output to avoid distracting the user 602).

### Example Augmented Reality Interaction

Figure 6B shows the user 602 wearing the wrist-wearable device 626 and the AR device 628 and holding the HIPD 642. In the second AR system 600b, the wrist-wearable device 626, the AR device 628, and/or the HIPD 642 are used to receive and/or provide one or more messages to a contact of the user 602. In particular, the wrist-wearable device 626, the AR device 628, and/or the HIPD 642 detect and coordinate one or more user inputs to initiate a messaging application and prepare a response to a received message via the messaging application.

In some embodiments, the user 602 initiates, via a user input, an application on the wrist-wearable device 626, the AR device 628, and/or the HIPD 642 that causes the application to initiate on at least one device. For example, in the second AR system 600b the user 602 performs a hand gesture associated with a command for initiating a messaging application (represented by messaging user interface 612); the wrist-wearable device 626 detects the hand gesture; and, based on a determination that the user 602 is wearing the AR device 628, causes the AR device 628 to present a messaging user interface 612 of the messaging application. The AR device 628 can present the messaging user interface 612 to the user 602 via its display (e.g., as shown by user 602's field of view 610). In some embodiments, the application is initiated and can be run on the device (e.g., the wrist-wearable device 626, the AR device 628, and/or the HIPD 642) that detects the user input to initiate the application, and the device provides another device operational data to cause the presentation of the messaging application. For example, the wrist-wearable device 626 can detect the user input to initiate a messaging application, initiate and run the messaging application, and provide operational data to the AR device 628 and/or the HIPD 642 to cause presentation of the messaging application. Alternatively, the application can be initiated and run at a device other than the device that detected the user input. For example, the wrist-wearable device 626 can detect the hand gesture associated with initiating the messaging application and cause the HIPD 642 to run the messaging application and coordinate the presentation of the messaging application.

Further, the user 602 can provide a user input provided at the wrist-wearable device 626, the AR device 628, and/or the HIPD 642 to continue and/or complete an operation initiated at another device. For example, after initiating the messaging application via the wrist-wearable device 626 and while the AR device 628 presents the messaging user interface 612, the user 602 can provide an input at the HIPD 642 to prepare a response (e.g., shown by the swipe gesture performed on the HIPD 642). The user 602's gestures performed on the HIPD 642 can be provided and/or displayed on another device. For example, the user 602's swipe gestures performed on the HIPD 642 are displayed on a virtual keyboard of the messaging user interface 612 displayed by the AR device 628.

In some embodiments, the wrist-wearable device 626, the AR device 628, the HIPD 642, and/or other communicatively coupled devices can present one or more notifications to the user 602. The notification can be an indication of a new message, an incoming call, an application update, a status update, etc. The user 602 can select the notification via the wrist-wearable device 626, the AR device 628, or the HIPD 642 and cause presentation of an application or operation associated with the notification on at least one device. For example, the user 602 can receive a notification that a message was received at the wrist-wearable device 626, the AR device 628, the HIPD 642, and/or other communicatively coupled device and provide a user input at the wrist-wearable device 626, the AR device 628, and/or the HIPD 642 to review the notification, and the device detecting the user input can cause an application associated with the notification to be initiated and/or presented at the wrist-wearable device 626, the AR device 628, and/or the HIPD 642.

While the above example describes coordinated inputs used to interact with a messaging application, the skilled artisan will appreciate upon reading the descriptions that user inputs can be coordinated to interact with any number of applications including, but not limited to, gaming applications, social media applications, camera applications, web-based applications, financial applications, etc. For example, the AR device 628 can present to the user 602 game application data and the HIPD 642 can use a controller to provide inputs to the game. Similarly, the user 602 can use the wrist-wearable device 626 to initiate a camera of the AR device 628, and the user can use the wrist-wearable device 626, the AR device 628, and/or the HIPD 642 to manipulate the image capture (e.g., zoom in or out, apply filters) and capture image data.

While an AR device 628 is shown being capable of certain functions, it is understood that an AR device can be an AR device with varying functionalities based on costs and market demands. For example, an AR device may include a single output modality such as an audio output modality. In another example, the AR device may include a low-fidelity display as one of the output modalities, where simple information (e.g., text and/or low-fidelity images/video) is capable of being presented to the user. In yet another example, the AR device can be configured with face-facing light emitting diodes (LEDs) configured to provide a user with information, e.g., an LED around the right-side lens can illuminate to notify the wearer to turn right while directions are being provided or an LED on the left side can illuminate to notify the wearer to turn left while directions are being provided. In another embodiment, the AR device can include an outward-facing projector such that information (e.g., text information, media) may be displayed on the palm of a user's hand or other suitable surface (e.g., a table, whiteboard). In yet another embodiment, information may also be provided by locally dimming portions of a lens to emphasize portions of the environment in which the user's attention should be directed. Some AR devices can present AR augments either monocularly or binocularly (e.g., an AR augment can be presented at only a single display associated with a single lens as opposed to presenting an AR augmented at both lenses to produce a binocular image). In some instances, an AR device capable of presenting AR augments binocularly can optionally display AR augments monocularly as well (e.g., for power-saving purposes or other presentation considerations). These examples are non-exhaustive and features of one AR device described above can be combined with features of another AR device described above. While features and experiences of an AR device have been described generally in the preceding sections, it is understood that the described functionalities and experiences can be applied in a similar manner to an MR headset, which is described below in the proceeding sections.

### Example Mixed Reality Interaction

Turning to Figures 6C-1 and 6C-2, the user 602 is shown wearing the wrist-wearable device 626 and an MR device 632 (e.g., a device capable of providing either an entirely VR experience or an MR experience that displays object(s) from a physical environment at a display of the device) and holding the HIPD 642. In the third AR system 600c, the wrist-wearable device 626, the MR device 632, and/or the HIPD 642 are used to interact within an MR environment, such as a VR game or other MR/VR application. While the MR device 632 presents a representation of a VR game (e.g., first MR game environment 620) to the user 602, the wrist-wearable device 626, the MR device 632, and/or the HIPD 642 detect and coordinate one or more user inputs to allow the user 602 to interact with the VR game.

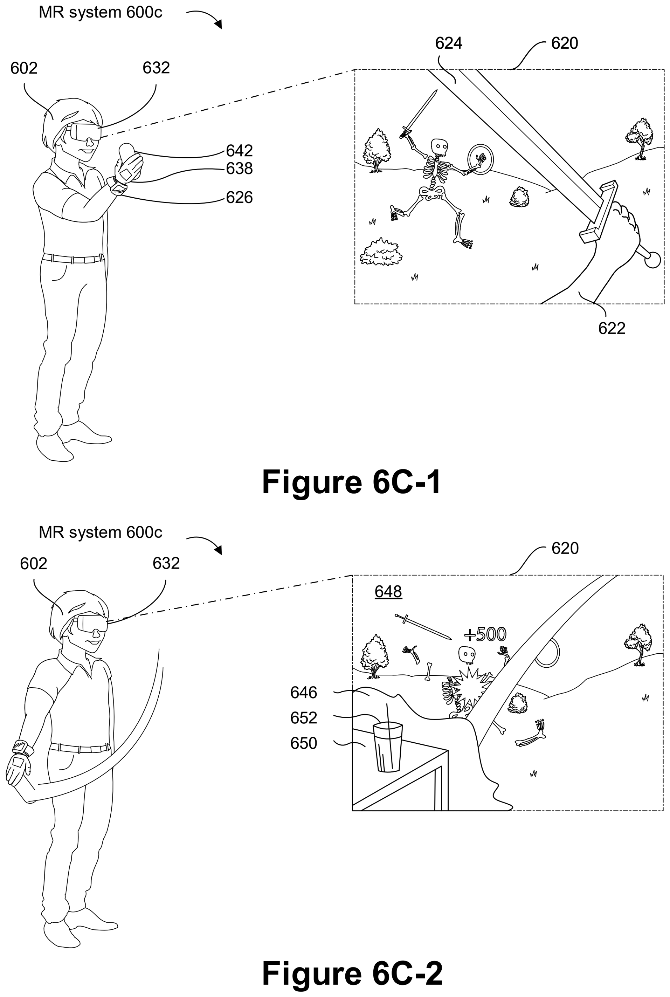

In some embodiments, the user 602 can provide a user input via the wrist-wearable device 626, the MR device 632, and/or the HIPD 642 that causes an action in a corresponding MR environment. For example, the user 602 in the third MR system 600c (shown in Figure 6C-1) raises the HIPD 642 to prepare for a swing in the first MR game environment 620. The MR device 632, responsive to the user 602 raising the HIPD 642, causes the MR representation of the user 622 to perform a similar action (e.g., raise a virtual object, such as a virtual sword 624). In some embodiments, each device uses respective sensor data and/or image data to detect the user input and provide an accurate representation of the user 602's motion. For example, image sensors (e.g., SLAM cameras or other cameras) of the HIPD 642 can be used to detect a position of the HIPD 642 relative to the user 602's body such that the virtual object can be positioned appropriately within the first MR game environment 620; sensor data from the wrist-wearable device 626 can be used to detect a velocity at which the user 602 raises the HIPD 642 such that the MR representation of the user 622 and the virtual sword 624 are synchronized with the user 602's movements; and image sensors of the MR device 632 can be used to represent the user 602's body, boundary conditions, or real-world objects within the first MR game environment 620.

In Figure 6C-2, the user 602 performs a downward swing while holding the HIPD 642. The user 602's downward swing is detected by the wrist-wearable device 626, the MR device 632, and/or the HIPD 642 and a corresponding action is performed in the first MR game environment 620. In some embodiments, the data captured by each device is used to improve the user's experience within the MR environment. For example, sensor data of the wrist-wearable device 626 can be used to determine a speed and/or force at which the downward swing is performed and image sensors of the HIPD 642 and/or the MR device 632 can be used to determine a location of the swing and how it should be represented in the first MR game environment 620, which, in turn, can be used as inputs for the MR environment (e.g., game mechanics, which can use detected speed, force, locations, and/or aspects of the user 602's actions to classify a user's inputs (e.g., user performs a light strike, hard strike, critical strike, glancing strike, miss) or calculate an output (e.g., amount of damage)).

Figure 6C-2 further illustrates that a portion of the physical environment is reconstructed and displayed at a display of the MR device 632 while the MR game environment 620 is being displayed. In this instance, a reconstruction of the physical environment 646 is displayed in place of a portion of the MR game environment 620 when object(s) in the physical environment are potentially in the path of the user (e.g., a collision with the user and an object in the physical environment are likely). Thus, this example MR game environment 620 includes (i) an immersive VR portion 648 (e.g., an environment that does not have a corollary counterpart in a nearby physical environment) and (ii) a reconstruction of the physical environment 646 (e.g., table 650 and cup 652). While the example shown here is an MR environment that shows a reconstruction of the physical environment to avoid collisions, other uses of reconstructions of the physical environment can be used, such as defining features of the virtual environment based on the surrounding physical environment (e.g., a virtual column can be placed based on an object in the surrounding physical environment (e.g., a tree)).

While the wrist-wearable device 626, the MR device 632, and/or the HIPD 642 are described as detecting user inputs, in some embodiments, user inputs are detected at a single device (with the single device being responsible for distributing signals to the other devices for performing the user input). For example, the HIPD 642 can operate an application for generating the first MR game environment 620 and provide the MR device 632 with corresponding data for causing the presentation of the first MR game environment 620, as well as detect the user 602's movements (while holding the HIPD 642) to cause the performance of corresponding actions within the first MR game environment 620. Additionally or alternatively, in some embodiments, operational data (e.g., sensor data, image data, application data, device data, and/or other data) of one or more devices is provided to a single device (e.g., the HIPD 642) to process the operational data and cause respective devices to perform an action associated with processed operational data.

In some embodiments, the user 602 can wear a wrist-wearable device 626, wear an MR device 632, wear smart textile-based garments 638 (e.g., wearable haptic gloves), and/or hold an HIPD 642 device. In this embodiment, the wrist-wearable device 626, the MR device 632, and/or the smart textile-based garments 638 are used to interact within an MR environment (e.g., any AR or MR system described above in reference to Figures 6A-6B). While the MR device 632 presents a representation of an MR game (e.g., second MR game environment 620) to the user 602, the wrist-wearable device 626, the MR device 632, and/or the smart textile-based garments 638 detect and coordinate one or more user inputs to allow the user 602 to interact with the MR environment.

In some embodiments, the user 602 can provide a user input via the wrist-wearable device 626, an HIPD 642, the MR device 632, and/or the smart textile-based garments 638 that causes an action in a corresponding MR environment. In some embodiments, each device uses respective sensor data and/or image data to detect the user input and provide an accurate representation of the user 602's motion. While four different input devices are shown (e.g., a wrist-wearable device 626, an MR device 632, an HIPD 642, and a smart textile-based garment 638) each one of these input devices entirely on its own can provide inputs for fully interacting with the MR environment. For example, the wrist-wearable device can provide sufficient inputs on its own for interacting with the MR environment. In some embodiments, if multiple input devices are used (e.g., a wrist-wearable device and the smart textile-based garment 638) sensor fusion can be utilized to ensure inputs are correct. While multiple input devices are described, it is understood that other input devices can be used in conjunction or on their own instead, such as but not limited to external motion-tracking cameras, other wearable devices fitted to different parts of a user, apparatuses that allow for a user to experience walking in an MR environment while remaining substantially stationary in the physical environment, etc.

As described above, the data captured by each device is used to improve the user's experience within the MR environment. Although not shown, the smart textile-based garments 638 can be used in conjunction with an MR device and/or an HIPD 642.

While some experiences are described as occurring on an AR device and other experiences are described as occurring on an MR device, one skilled in the art would appreciate that experiences can be ported over from an MR device to an AR device, and vice versa.

Some definitions of devices and components that can be included in some or all of the example devices discussed are defined here for ease of reference. A skilled artisan will appreciate that certain types of the components described may be more suitable for a particular set of devices, and less suitable for a different set of devices. But subsequent reference to the components defined here should be considered to be encompassed by the definitions provided.

In some embodiments example devices and systems, including electronic devices and systems, will be discussed. Such example devices and systems are not intended to be limiting, and one of skill in the art will understand that alternative devices and systems to the example devices and systems described herein may be used to perform the operations and construct the systems and devices that are described herein.

As described herein, an electronic device is a device that uses electrical energy to perform a specific function. It can be any physical object that contains electronic components such as transistors, resistors, capacitors, diodes, and integrated circuits. Examples of electronic devices include smartphones, laptops, digital cameras, televisions, gaming consoles, and music players, as well as the example electronic devices discussed herein. As described herein, an intermediary electronic device is a device that sits between two other electronic devices, and/or a subset of components of one or more electronic devices and facilitates communication, and/or data processing and/or data transfer between the respective electronic devices and/or electronic components.

The foregoing descriptions of Figures 6A-6C-2 provided above are intended to augment the description provided in reference to Figures 1-5. While terms in the following description may not be identical to terms used in the foregoing description, a person having ordinary skill in the art would understand these terms to have the same meaning.

Any data collection performed by the devices described herein and/or any devices configured to perform or cause the performance of the different embodiments described above in reference to any of the Figures, hereinafter the "devices," is done with user consent and in a manner that is consistent with all applicable privacy laws. Users are given options to allow the devices to collect data, as well as the option to limit or deny collection of data by the devices. A user is able to opt in or opt out of any data collection at any time. Further, users are given the option to request the removal of any collected data.

It will be understood that, although the terms "first," "second," etc. may be used herein to describe various elements, these elements should not be limited by these terms. These terms are only used to distinguish one element from another.

The terminology used herein is for the purpose of describing particular embodiments only and is not intended to be limiting of the claims. As used in the description of the embodiments and the appended claims, the singular forms "a," "an" and "the" are intended to include the plural forms as well, unless the context clearly indicates otherwise. It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" can be construed to mean "when" or "upon" or "in response to determining" or "in accordance with a determination" or "in response to detecting," that a stated condition precedent is true, depending on the context. Similarly, the phrase "if it is determined [that a stated condition precedent is true]" or "if [a stated condition precedent is true]" or "when [a stated condition precedent is true]" can be construed to mean "upon determining" or "in response to determining" or "in accordance with a determination" or "upon detecting" or "in response to detecting" that the stated condition precedent is true, depending on the context.

The foregoing description, for the purpose of explanation, has been described with reference to specific embodiments. However, the illustrative discussions above are not intended to be exhaustive or to limit the claims to the precise forms disclosed. Many modifications and variations are possible in view of the above teachings. The embodiments were chosen and described in order to best explain principles of operation and practical applications, to thereby enable others skilled in the art.

## Claims

1. A wrist-wearable device, comprising:
a wearable capsule, wherein the wearable capsule includes a bottom capsule portion configured to contact a user;
an electromyography, EMG, sensor disposed within the wearable capsule, the EMG sensor configured to provide biopotential information of a user for determining a movement of the user; and
a photoplethysmogram, PPG, sensor disposed within the wearable capsule, the PPG sensor configured to provide information for determining a heart rate of the user, wherein the biopotential information and information for determining the heart rate of the user is received simultaneously, and sensing of the biopotential information does not alter the information for determining heart rate of the user, and sensing of the information for determining the heart rate of the user does not alter the biopotential information.

2. The wrist-wearable device of claim 1, further comprising:
a plurality of electrodes coupled to the bottom capsule portion and electrically connected to the EMG sensor, wherein the plurality of electrodes is configured to permit transmission of biopotential information from the user to the EMG sensor, and
optionally wherein the plurality of electrodes is integrated with the bottom capsule portion.

3. The wrist-wearable device of claim 2, wherein a central portion of the bottom capsule portion coupled to the plurality of electrodes is raised relative to a surrounding portion of the bottom capsule portion.

4. The wrist-wearable device of claim 2 or claim 3, wherein the plurality of electrodes is sealingly coupled to the bottom capsule portion.

5. The wrist-wearable device of claim 4, further comprising a sealing gasket disposed between an electrode of the plurality of electrodes and the bottom capsule portion.

6. The wrist-wearable device of any of claims 2 to 5, further comprising a printed circuit board disposed within the wearable capsule, wherein the EMG sensor and the PPG sensor are coupled to the printed circuit board, and
optionally further comprising a fastener extending through the printed circuit board removably coupled to an electrode of the plurality of electrodes, wherein the fastener is configured to couple the printed circuit board to the wearable capsule.

7. The wrist-wearable device of claim 6, wherein the printed circuit board includes power line interference shielding.

8. The wrist-wearable device of any preceding claim, the wrist-wearable device further comprising:
a plurality of light pipes coupled to the PPG sensor and the bottom capsule portion, wherein the plurality of light pipes is configured to permit transmission of light between the PPG sensor and the bottom capsule portion, and
optionally wherein the plurality of light pipes is integrated with the bottom capsule portion.

9. The wrist-wearable device of claim 8, wherein the plurality of light pipes includes an outlet light pipe configured to transmit light from an outlet portion of PPG sensor to the user and an inlet light pipe configured to transmit light from the user to an inlet portion of the PPG sensor, and
optionally wherein the wrist-wearable device further comprises:
an optical sealing gasket disposed between the outlet portion of the PPG sensor and the inlet portion of the PPG sensor, wherein the optical sealing gasket is configured to reduce crosstalk between the outlet portion and the inlet portion, and further optionally wherein the optical sealing gasket includes:
a compressible foam layer; and
a pressure-sensitive adhesive layer coupled to the compressible foam layer.

10. The wrist-wearable device of any preceding claim, wherein the bottom capsule portion comprises polycarbonate or polybutylene terephthalate.

11. A wrist-wearable device, comprising:
a wearable capsule, wherein the wearable capsule includes a bottom capsule portion configured to contact a user;
an electromyography, EMG, sensor disposed within the wearable capsule, the EMG sensor configured to provide biopotential information of a user for determining a movement of the user;
a plurality of electrodes coupled to the bottom capsule portion and electrically connected to the EMG sensor, wherein the plurality of electrodes is configured to permit transmission of biopotential information from the user to the EMG sensor;
a photoplethysmogram, PPG, sensor disposed within the wearable capsule, the PPG sensor configured to provide information for determining a heart rate of the user; and
a plurality of light pipes coupled to the PPG sensor and the bottom capsule portion, wherein the plurality of light pipes is configured to permit the transmission of light between the PPG sensor and the bottom capsule portion,
wherein the biopotential information and information for determining the heart rate of the user is received simultaneously, and sensing of the biopotential information does not alter the information for determining heart rate of the user, and sensing of the information for determining the heart rate of the user does not alter the biopotential information.

12. The wrist-wearable device of claim 11, wherein the plurality of electrodes and the plurality of light pipes are each integrated with the bottom capsule portion.

13. The wrist-wearable device of claim 11 or claim 12, further comprising:
an optical sealing gasket comprising:
a compressible foam layer; and
a pressure-sensitive adhesive layer coupled to the compressible foam layer, wherein the optical sealing gasket is disposed between an outlet portion of the PPG sensor and an inlet portion of the PPG sensor,
wherein the optical sealing gasket is configured to reduce crosstalk between the outlet portion and the inlet portion and reduce compressive force applied to the plurality of electrodes.

14. A method to manufacture a wrist-wearable device, the method comprising:
injecting a first material into a mold to form a first shot of a bottom capsule portion of the wrist-wearable device, wherein the first shot of the bottom capsule portion includes a plurality of light pipes configured to permit transmission of light between a photoplethysmogram, PPG, sensor and the bottom capsule portion;
positioning a plurality of electrodes relative to the first shot of the bottom capsule portion of the wrist-wearable device, wherein the plurality of electrodes is configured to permit transmission of biopotential information from a user to an electromyography, EMG, sensor; and
injecting a second material into the mold to form a second shot of the bottom capsule portion of the wrist-wearable device, wherein the second shot of the bottom capsule portion bonds the plurality of electrodes to the bottom capsule portion of the wrist-wearable device.

15. The method of claim 14, further comprising:
applying a hard coat to the bottom capsule portion and the plurality of electrodes; and
removing the hard coat from the plurality of electrodes after applying the hard coat to the bottom capsule portion.
